# EUROPEAN PATENT APPLICATION

(11) **EP 4 385 619 A1**
(43) Date of publication of application: **19.06.2024**
(21) Application number: 22213597.2
(22) Date of filing: 14.12.2022
(51) Int. Cl.: B01J 21/04, C07C 5/00, B01J 23/36, B01J 35/08, B01J 37/02

(54) **METHODS OF PREPARATION AND USES OF ALKALI METAL OR RHENIUM MODIFIED ALUMINA CATALYSTS**

(71) Applicant: SABIC Global Technologies B.V., 4612 PX Bergen op Zoom (NL)
(72) Inventor: Guggilla, Vidya Sagar, 562125 Bengaluru (IN); Subramanian, Selvakumar, 562125 Bengaluru (IN); Alkhudeer, Saud, 562125 Bengaluru (IN)
(74) Representative: Sabic INDIA Intellectual Property Group

(57) **Abstract**

Provided here are alkali metal or rhenium modified alumina catalysts, methods of their preparation, and methods for their use in double bond isomerization of aliphatic light internal olefins to aliphatic α-olefins and in reverse isomerization reactions.

## Description

### Technical Field

The disclosure relates to alkali metal or rhenium modified alumina catalysts, methods of their preparation, and methods for their use in isomerization reactions.

### Background

A co-monomer for the production of linear low-density polyethylene and polybutene is 1-butene. And, along with or without 2-butene, 1-butene is converted to produce propylene or linear olefins by metathesis reaction based on product requirement. Currently, 1-butene is produced through separation processes from C4 fractions in the naphtha cracking in a petrochemical plant. When these processes are completed, C4 raffinate-III, which contains much 2-butene, remains. Therefore, it is necessary to convert 2-butene into 1-butene with higher added values through reverse /positional isomerization. There are other possibilities of generating isobutene through skeletal isomerization, octene and dodecene through oligomerization, and other light hydrocarbons through cracking over a catalyst. More importantly, the utilized catalysts still suffer from low selectivity of 1-butene and fast deactivation. There is commercial co-monomer production by isomerization of 2-butene of raffinate-III to produce 1-butene and followed by metathesis to produce 1-hexene in the presence of a magnesium oxide catalyst. However, this catalyst deactivates rapidly and is very sensitive to moisture.

### Summary

Applicant has identified a necessity for the development of highly selective and stable catalyst for isomerization reactions of aliphatic light internal olefins to aliphatic α-olefins. Provided here are compositions and methods to address these shortcomings of the art and provide other additional or alternative advantages. The disclosure herein provides several embodiments of methods for preparation of potassium or rhenium modified alumina catalysts and methods for use of these resulting compositions in isomerization reactions.

Embodiments include methods for olefin bond isomerization in the presence of a γ-alumina-based catalyst. One such method includes the steps of heating a γ-alumina-based catalyst in an isomerization reactor to a temperature ranging from about 450 °C to about 550 °C to produce an activated γ-alumina-based catalyst, cooling the activated γ-alumina-based catalyst to a temperature below 450°C, and supplying an aliphatic light internal olefin-rich stream to the isomerization reactor to convert a portion of the aliphatic light internal olefin-rich stream to an aliphatic α-olefin-rich stream. The activated γ-alumina-based catalyst is cooled in the presence of nitrogen. The aliphatic light internal olefin-rich stream can contain greater than 30 wt. % of 2-butenes. In certain embodiments, the activated γ-alumina-based catalyst contains either an alkali metal hydroxide ranging from 5 weight percent (wt.%) to 10 wt.% or rhenium oxide ranging from 3 wt.% to 8 wt.%. It has surprisingly been discovered that activated γ-alumina-based catalyst containing either an alkali metal hydroxide ranging from 5 weight percent (wt.%) to 10 wt.% or rhenium oxide ranging from 3 wt.% to 8 wt.% exhibits increased stability and longer durations of activity before the catalyst becomes deactivated. In certain embodiments, the activated γ-alumina-based catalyst has a surface area ranging from about 175 to 250 square meters per gram. In certain embodiments, the alkali metal hydroxide is a potassium hydroxide. In certain embodiments, the isomerization reactor is a tubular fixed bed reactor. In certain embodiments, the method may be a method for the isomerization of 2-butene to 1-butene.

Embodiments include methods for preparing a γ-alumina-based catalyst. One such method includes the steps of calcining a γ-alumina-based support at a temperature ranging from about 450 °C to about 550 °C to produce a calcined γ-alumina-based support, treating the calcined γ-alumina-based support with an aqueous alkali metal hydroxide solution to form an impregnated γ-alumina-based support, drying the impregnated γ-alumina-based a support at a temperature ranging from about 80 °C to about 120 °C, and calcining the impregnated alumina support at a temperature ranging from about 450 °C to about 550 °C to produce the γ-alumina-based catalyst. The aqueous alkali metal hydroxide solution can be sprayed over the γ-alumina-based support in a rotary-type impregnator unit. The aqueous alkali metal hydroxide solution can be sprayed over the γ-alumina-based support in the rotary-type impregnator unit at a gauge pressure of about 1.5 barg to about 4 barg. The amount of the alkali metal hydroxide in the γ-alumina-based catalyst ranges from 5 wt.% to 10 wt.%. In certain embodiments, the aqueous alkali metal hydroxide solution contains potassium hydroxide. The aqueous alkali metal hydroxide solution can contain potassium hydroxide with a molar concentration ranging from about 4M to about 8M. In certain embodiments, the γ-alumina-based support contains γ-alumina-based spheres with a surface area ranging from about 175 m²/g to about 250 m²/g. In certain embodiments, the γ-alumina-based support contains γ-alumina-based spheres with a pore volume ranging from about 0.5 milliliter per gram (mL/g) to about 0.75 mL/g.

Another method for preparing a γ-alumina-based catalyst includes the steps of calcining a γ-alumina-based support at a temperature ranging from about 450 °C to about 550 °C to produce a calcined γ-alumina-based support, treating the calcined γ-alumina-based support with an aqueous perrhenic acid solution to form an impregnated γ-alumina-based support, drying the impregnated γ-alumina-based a support at a temperature ranging from about 80 °C to about 120 °C, and calcining the impregnated alumina support at a temperature ranging from about 450 °C to about 550 °C to produce the γ-alumina-based catalyst. The aqueous perrhenic acid solution can be sprayed over the γ-alumina-based support in a rotary-type impregnator unit. The aqueous perrhenic acid solution can be sprayed over the γ-alumina-based support in the rotary-type impregnator unit at a gauge pressure of about 2 barg. The γ-alumina-based catalyst can contain rhenium oxide ranging from 3 wt.% to 8 wt.%. In certain embodiments, the γ-alumina-based support contains γ-alumina-based spheres with a surface area ranging from about 175 m²/g to about 250 m²/g. In certain embodiments, the γ-alumina-based support contains γ-alumina-based spheres with a pore volume ranging from about 0.5 mL/g to about 0.75 mL/g.

### Brief Description of the Drawings

Embodiments will be readily understood by the following detailed description in conjunction with the accompanying drawings. To facilitate this description, like reference numerals designate like structural elements or procedures in a method. Embodiments are illustrated by way of example and not by way of limitation in the accompanying drawings. The present disclosure can be better understood by referring to the following figures. These drawings illustrate the principles of the disclosure and no limitation of the scope of the disclosure is thereby intended.
**FIG. 1** is a diagrammatic representation of a method for olefin bond isomerization in the presence of a γ-alumina-based catalyst, according to an embodiment.
**FIG. 2** is a diagrammatic representation of a method for preparing a K/γ-Al₂O₃ catalyst, according to an embodiment.
**FIG. 3** is a diagrammatic representation of a method for preparing a Re₂O₇/γ-Al₂O₃ catalyst, according to an embodiment.
**FIG. 4** is a graphical representation of the rigor and reproducibility of the catalyst for butene isomerization reaction as a function of time on stream, according to an embodiment.
**FIG. 5** is a graphical representation of the production of 1-butene at different reaction conditions using the K/γ-Al₂O₃ catalyst, according to an embodiment.
**FIG. 6** is a graphical representation of the production of 1-butene using 5 wt. % Re₂O₇/γ-Al₂O₃ under the reaction conditions, according to an embodiment.

### Detailed Description

The present disclosure describes various embodiments related to alkali metal alumina catalyst compositions and methods for of use in isomerization reactions. Certain embodiments include isomerization reactions of aliphatic light internal olefins to aliphatic α-olefins.

Embodiments of the alkali metal modified-alumina catalysts or rhenium modified-alumina catalysts. Methods of preparation of these catalysts include the steps of calcining a γ-alumina support, treating the γ-alumina support with a solution of an alkali metal hydroxide (such as KOH) or a perrhenic acid (HReO4) to form an impregnated alumina support containing K/y-Al₂O₃ or Re₂O₇/Al₂O₃, and drying and calcining the impregnated alumina support to form the reverse catalyst. In certain embodiments, the impregnated alumina support contains about 5 weight percent (wt.%) to about 10 wt.% of potassium hydroxide impregnated on the γ-Al₂O₃ support. In certain embodiments, the impregnated alumina support contains about 8 wt.% of potassium hydroxide impregnated on the γ-Al₂O₃ support. In certain embodiments, the impregnated alumina support contains about 9.5 wt.% of potassium hydroxide impregnated on the γ-Al₂O₃ support. In certain embodiments, the impregnated alumina support contains about 3 wt.% to about 8 wt.% of rhenium oxide impregnated on the γ-Al₂O₃ support. In certain embodiments, the impregnated alumina support contains about 5 wt.% of rhenium impregnated on the γ-Al₂O₃ support.

Embodiments of uses of the foregoing catalysts in the isomerization of aliphatic light internal olefins to aliphatic α-olefins.Methods of use of these catalysts in the isomerization reaction involve activation and cooling of the catalysts prior to introduction of the aliphatic light internal olefins at the reaction temperature. Certain embodiments include the processing of 2-butene by supplying it to a reverse isomerization reactor containing an alkali metal alumina catalyst to produce a 1-butene-rich stream. These catalysts exhibit high selectivity to the foregoing 1-butene production.

In the following description, reference is made to the accompanying drawings that form a part of this disclosure and numerous details are set forth in order to provide a thorough understanding of the various embodiments. In other instances, well-known processes, devices, and systems may not been described in particular detail in order not to unnecessarily obscure the various embodiments. Additionally, illustrations of the various embodiments may omit certain features or details in order to not obscure the various embodiments. The drawings may provide an illustration of some of the various embodiments in which the subject matter of the present disclosure may be practiced. Other embodiments may be utilized, and logical changes may be made without departing from the scope of this disclosure.

The description may use the phrases "in some embodiments," "in various embodiments," "in an embodiment," or "in embodiments," which may each refer to one or more of the same or different embodiments. The words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The term "about" refers to a range of values including the specified value, which a person of ordinary skill in the art would consider reasonably similar to the specified value. In embodiments, "about" refers to values within a standard deviation using measurements generally acceptable in the art.. In one non-limiting embodiment, when the term "about" is used with a particular value, then "about" refers to a range extending to ±10% of the specified value, alternatively ±5% of the specified value, or alternatively ±1% of the specified value, or alternatively ±0.5% of the specified value. In embodiments, "about" refers to the specified value.

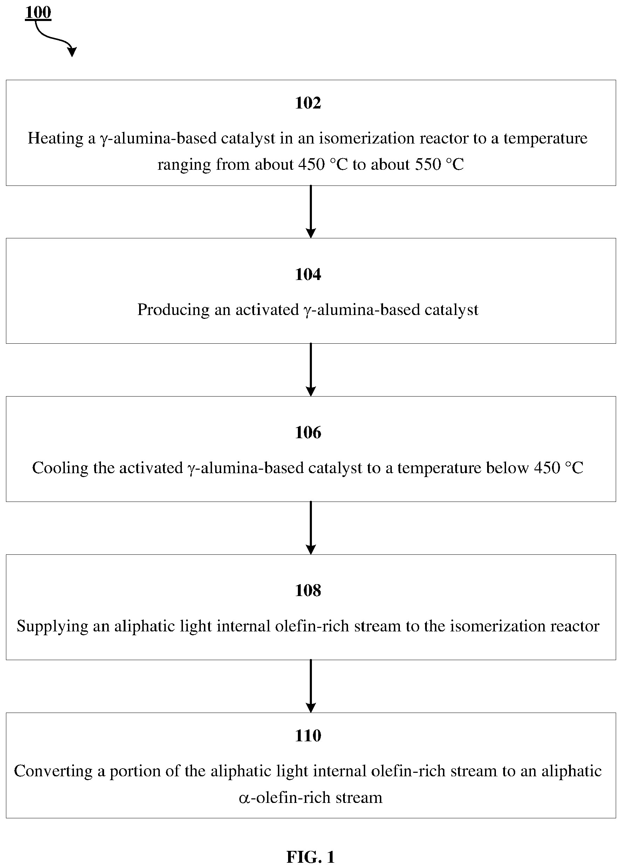

**FIG. 1** is a diagrammatic representation of a method **100** for olefin bond isomerization in the presence of a γ-alumina-based catalyst, according to an embodiment. The method **100** includes a step **102** of heating a γ-alumina-based catalyst in an isomerization reactor to a temperature ranging from about 450 °C to about 550 °C. The method includes the step **104** of producing and extracting an activated γ-alumina-based catalyst. In certain embodiments, the activated γ-alumina-based catalyst contains either an alkali metal hydroxide ranging from 5 wt.% to 10 wt.% or rhenium oxide ranging from 3 wt.% to 8 wt.%. The step **106** of the method includes cooling the activated γ-alumina-based catalyst to a temperature below 450°C. The activated γ-alumina-based catalyst is cooled in the presence of nitrogen. At step **108**, an aliphatic light internal olefin-rich stream is supplied to the isomerization reactor, and at step 110, a portion of the aliphatic light internal olefin-rich stream is converted to an aliphatic α-olefin-rich stream. In certain embodiments, the aliphatic α-olefin-rich stream is supplied to a separation unit to purify the aliphatic α-olefin.

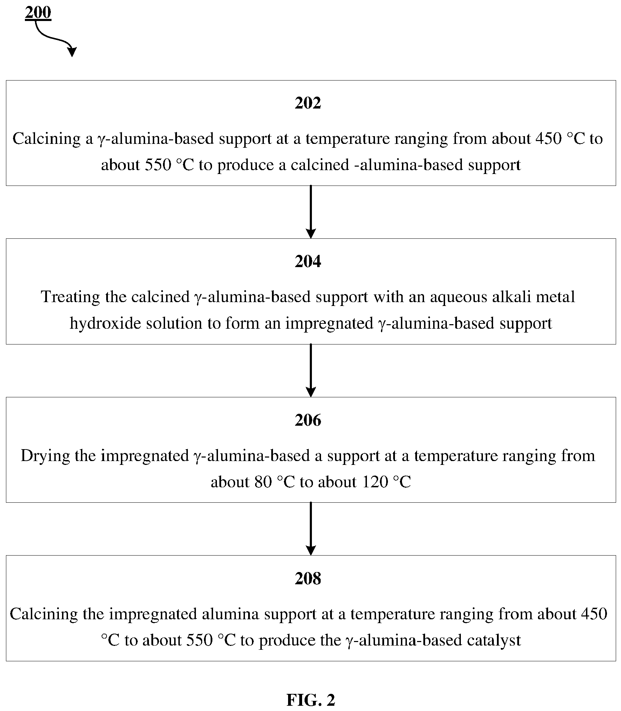

**FIG. 2** is a diagrammatic representation of a method **200** for preparing a K/γ-Al₂O₃ catalyst, according to an embodiment. The method **200** includes a step **202** of calcining a γ-alumina-based support at a temperature ranging from about 450 °C to about 550 °C to produce a calcined γ-alumina-based support. The step **204** involves treating the calcined γ-alumina-based support with an aqueous alkali metal hydroxide solution to form an impregnated γ-alumina-based support. The step **206** involves drying the impregnated γ-alumina-based a support at a temperature ranging from about 80 °C to about 120 °C. The step **208** involves calcining the impregnated alumina support at a temperature ranging from about 450 °C to about 550 °C to produce the γ-alumina-based catalyst.

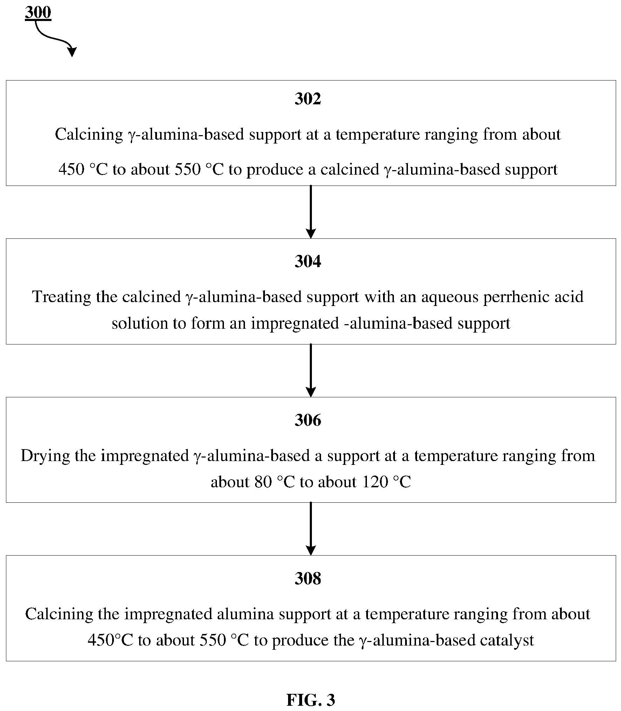

**FIG. 3** is a diagrammatic representation of a method **300** for preparing a Re₂O₇/γ-Al₂O₃ catalyst, according to an embodiment. The method **300** includes a step 302 of calcining a γ-alumina-based support at a temperature ranging from about 450 °C to about 550 °C to produce a calcined γ-alumina-based support. The step **304** involves treating the calcined γ-alumina-based support with an aqueous perrhenic acid solution to form an impregnated γ-alumina-based support. The step **306** involves drying the impregnated γ-alumina-based a support at a temperature ranging from about 80 °C to about 120 °C. The method **300** includes a step 308 of calcining the impregnated alumina support at a temperature ranging from about 450 °C to about 550 °C to produce the γ-alumina-based catalyst.

Methods of preparation of these catalysts include the steps of calcining a γ-alumina support, treating the γ-alumina support with a solution of an alkali metal hydroxide (such as KOH) or a perrhenic acid (HReO4) to form an impregnated alumina support containing K/γ-Al₂O₃ or Re₂O₇/γ-Al₂O₃, and drying and calcining the impregnated alumina support to form the reverse catalyst. In certain embodiments, the impregnated alumina support contains about 5 weight percent (wt.%) to about 10 wt.% of potassium hydroxide impregnated on the γ-Al₂O₃ support. In certain embodiments, the impregnated alumina support contains about 3 wt.% to about 8 wt.% of rhenium oxide impregnated on the γ-Al₂O₃ support. In certain embodiments, the γ-alumina support is in the form of spheres. In certain embodiments, the γ-alumina support is subject to calcination at a temperature ranging from about 500 °C to about 600 °C. Calcination of the γ-alumina support prior to the impregnation step facilitates the removal of moisture to ensure pore volume availability for the impregnation step with an alkali metal hydroxide (such as KOH) or a perrhenic acid (HReO4) solution. Calcination of the impregnated alumina support leads to the loss of water and production of the oxide form of the alkali metal hydroxide (such as KOH) or a perrhenic acid on the alumina, such as K/γ-Al₂O₃ or Re₂O₇/γ-Al₂O₃ to establish metal-support interaction.

Embodiment of the alumina support include crystalline phase alumina, such as γ-Al₂O₃ and θ-Al₂O₃. The surface area of the alumina support ranges from about 175 square meters per gram (m²/g) to about 250 m²/g. The pore volume of the alumina support ranges from about 0.5 milliliters per gram (mL/g) to about 0.75 mL/g. The pore diameter of the alumina support ranges from about 70 Angstroms (Å) to about 80 Å. The bulk density of the alumina support ranges from about 0.4 grams per milliliter (g/mL) to about 0.8 g/mL. The mean diameter of the alumina support in the form of spherical pellets ranges from about 1.2 millimeter (mm) to about 3 mm. The mean diameter of the alumina support in the form of extrudates/trilobes/quadralobes ranges from about 1.6 mm to 2 mm. The crush strength of the alumina support in the form of spherical pellets ranges from about 4 decaNewton per millimeter (daN/mm) to about 15 daN/mm. The crush strength of the alumina support in the form of extrudates or tri-lobes ranges from about 2 daN/mm to about 4 daN/mm. In certain embodiments, commercially available γ-Al₂O₃ spheres with the following properties were procured: an average particle size of 1.2 mm to 2 mm, a surface area of 200 m²/g to 225 m²/g, a pore volume of 0.5 ml/g to 0.65 ml/g, a bulk density of 0.65 g/ml to 0.7 g/ml, a particle crush strength of 8 daN to 12 daN and a loss on ignition of less than 5 wt.%.

Embodiments include use of the alkali metal modified-alumina catalysts or rhenium modified-alumina catalysts for the double bond isomerization of aliphatic light internal olefins to aliphatic α-olefins, such as the conversion of 2-butene to 1-butene by reverse positional isomerization. One such method for olefin bond isomerization in the presence of a γ-alumina-based catalyst includes the steps of: heating a γ-alumina-based catalyst in an isomerization reactor to a temperature ranging from about 450 °C to about 550 °C to produce an activated γ-alumina-based catalyst, and cooling the activated γ-alumina-based catalyst to a temperature below 450°C, and supplying an aliphatic light internal olefin-rich stream to the isomerization reactor to convert a portion of the aliphatic light internal olefin-rich stream to an aliphatic α-olefin-rich stream. The activated γ-alumina-based catalyst contains either an alkali metal hydroxide ranging from 5 weight percent (wt.%) to 10 wt.% or rhenium oxide ranging from 3 wt.% to 8 wt.%. In certain embodiments, the conversion of the aliphatic light internal olefin-rich stream to the aliphatic α-olefin-rich stream is performed at a temperature ranging from about 200 °C to about 250 °C and a pressure ranging from about 0 barg to about 30 barg.

Embodiments include use of the alkali metal modified-alumina catalysts or rhenium modified-alumina catalysts for the isomerization of aliphatic α-olefins to aliphatic light internal olefins, such as the conversion of 1-butene to 2-butene. One such method for olefin bond isomerization in the presence of a γ-alumina-based catalyst includes the steps of: heating a γ-alumina-based catalyst in an isomerization reactor to a temperature ranging from about 450 °C to about 550 °C to produce an activated γ-alumina-based catalyst, and cooling the activated γ-alumina-based catalyst to a temperature below 450°C, and supplying an aliphatic α-olefin-rich stream to the isomerization reactor to convert a portion of the aliphatic α-olefin-rich stream to an aliphatic light internal olefin-rich stream. The activated γ-alumina-based catalyst contains either an alkali metal hydroxide ranging from 5 weight percent (wt.%) to 10 wt.% or rhenium oxide ranging from 3 wt.% to 8 wt.%. In certain embodiments, the conversion of the aliphatic α-olefin-rich stream to the aliphatic light internal olefin-rich stream is performed at a temperature ranging from about 25 °C to about 100 °C and a pressure ranging from about 0 barg to about 30 barg.

In certain embodiments, the alkali metal modified-alumina catalysts or rhenium modified-alumina catalysts are first activated at a temperature higher than the reaction temperature before the temperature is reduced to the reaction temperature under nitrogen. For example, the alkali metal modified-alumina catalysts or rhenium modified-alumina catalysts are subject to an activation process at a temperature ranging from about 450 °C to about 550 °C under air to remove the moisture from catalyst for activation or regeneration of catalyst. This step ensures the exposure of the active catalytic sites to the reactant molecules for conversion. Moreover, this step also increases the cycle time of the catalyst. After this catalyst activation or regeneration step, in certain embodiments, the catalyst is purged with nitrogen to avoid contact between air & the hydrocarbons during the reaction time.

### EXAMPLES

Various examples provided herein illustrate selected aspects of the various embodiments of alkali metal modified-alumina catalysts or rhenium modified-alumina catalysts, methods of manufacture of these catalysts, and methods of use of these catalysts in isomerization and reverse isomerization reactions.

### Example 1

Potassium modified-alumina catalysts in the form of spherical pellets were prepared containing 8 wt.% K/γ-Al₂O₃. Commercially available γ-Al₂O₃ spheres with the following properties were procured: an average particle size of 1.2 mm to 2 mm, a surface area of 200 m²/g to 225 m²/g, a pore volume of 0.5 ml/g to 0.65 ml/g, a bulk density of 0.65 g/ml to 0.7 g/ml, a particle crush strength of 8 daN to 12 daN and a loss on ignition of less than 5 wt.%. The catalyst is prepared by incipient wetness impregnation of the support with an aqueous solution prepared by dissolving 2.7 grams of potassium hydroxide in 11 mL of DI water and made a clear solution. The impregnation was carried out by contacting the above prepared impregnation solution with 18.4 grams of calcined alumina sphere support at room temperature. The calcined support is introduced into a rotatable impregnation drum, which is revolved at 25 revolutions per minute and sprayed with a potassium containing impregnating solution. The potassium hydroxide solution has a concentration of ~4.4M. The amount of solution corresponds to the pore volume of the γ-alumina support, which is measured by the dropwise addition of demineralized water to 10 gm of γ-alumina support until the support is completely wet. The measured quantity of water, normalized to 1 gram of support, was 0.6 grams of water required to completely saturate 1 gram of alumina support. The ~4.4M KOH solution is sprayed with a pressure of 2 barg and at a rate of 39 mL per minute over the course of 12 minutes for 800 grams of the final calcined catalyst. This ~4.4M KOH solution is allowed to act on the support for further 5 minutes to 30 minutes to complete the impregnation step. The impregnated alumina support was then kept at room temperature for 2 hours and then dried at 120 °C for 16 hours. The dried sample was then calcined at 550 °C for 5 hours with heating rate of 5 °C /min in the present of air in down flow tubular reactor. After calcination, the catalyst was cooled in the presence of air and the catalyst was stored in an airtight container was used for isomerization of 2-butene to 1-butene. The final calculated composition of the catalyst is 8 wt. % K/Al₂O₃. This process was robust and scalable to prepare larger than lab scale amounts of catalyst. The synthesized catalyst properties are provided in Table 1.

**Table 1**

| | Support | K/Al₂O₃ Catalyst |
|---|---|---|
| BET surface area, m²/gm | 205 | 176.7 |
| Average Pore Volume, cm³/g | 0.524 | 0.444 |
| BJH desorption pore dia., Å | 79.3 | 77.1 |
| Crushing strength, daN | 7.56 | 5.6 |
| Bulk density, gm/mL | 0.666 | 0.717 |
| Attrition, % | 0.252 | 0.403 |

### Example 2

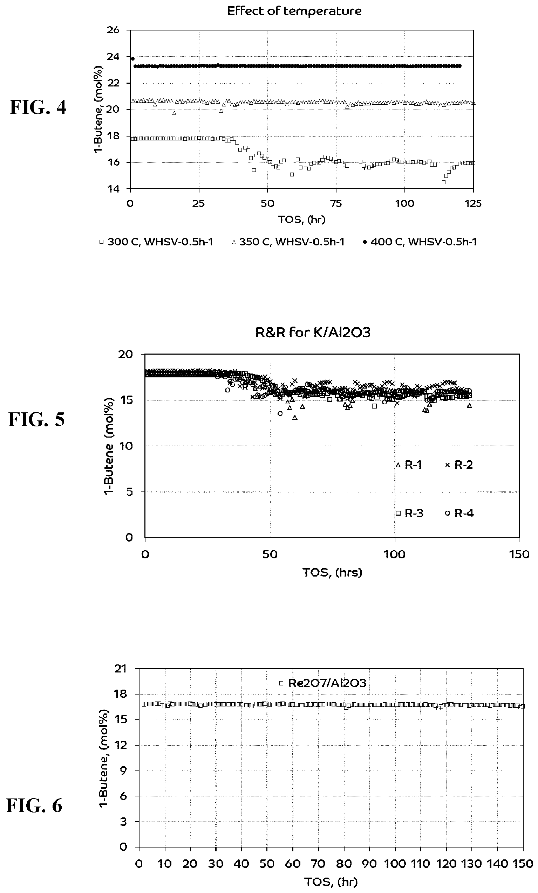

About 7 grams of catalyst spheres of mean diameter ranging from about 1.5 mm to about 2 mm, prepared as described in Example 1, were loaded into a tubular fixed bed reactor. The catalyst spheres were accurately weighted and loaded into the reactor. The plug flow conditions were maintained. The thermowell has been placed at bottom of the catalyst bed. A 13X molecular sieve was loaded above the catalyst bed to absorb the moisture from feedstock prior to contacting the catalyst bed. After loading, the catalyst was activated for the isomerization of 2-butene to 1-butene. Activation of the catalyst was performed under air at 550 °C for 6 hours, and then purging was carried out using air with nitrogen and cooled to 300 °C under nitrogen. Once a reaction temperature of 300 °C was reached, a feed containing 95% 2-butene and 5% iso-butane was supplied to the reactor. Iso-butane was used as an internal standard. The 2-butene feed was supplied to maintain a weight hourly space velocity (WHSV) of about 0.4/hr to 0.5/hr. The reactor outlet gases were analyzed by online gas chromatography (Agilent 6890) equipped with a flame ionization detector for hydrocarbon analysis and thermal conductivity detector for ideal gases. The reactor and products molar flowrates were calculated using internal standard iso-butane based on its molar flow rate as well as GC areas. FIG. 4 is a graphical representation of the rigor and reproducibility of the catalyst for butene isomerization reaction as a function of time on stream (TOS). There are no deactivation observed at different temperature tested for more than 100 hours of TOS.

### Example 3

The 8wt.% K/Al₂O₃ catalyst was tested at WHSV of 0.5/hr by varying reaction temperature at 300 °C, 350 °C, and 400 °C, and at atmospheric pressure. The activity results are shown in FIG. 5. The 1-butene formation increases with the increase of reaction temperature.

### Example 4

The catalyst process described in Example 1 was used to make 800 grams of the K/Al₂O₃ catalyst per batch size and about 10 kg of the K/Al₂O₃ catalyst was prepared in multiple batches. The catalyst prepared in an impregnator drum. The calcined spherical alumina was introduced into a rotatable impregnation drum. The drum was revolved at 25 revolutions per minute and sprayed with a potassium containing impregnating solution of ~4.4M. The KOH solution was sprayed with pressure of 2 barg and at a rate of 39 ml/min over the course of 12 minutes for 800 gm of final calcined catalyst. The impregnation solution is allowed to act on the support for a further 5 to 30 min after the impregnation. The impregnated support was dried at a temperature ranging from 80 to 120 °C for from 16 to 24 hours followed by calcination at 550 °C under air for 6 hours. And, the K/Al₂O₃ catalyst tested at pilot scale fixed bed reactor having a reactor with an internal diameter of 150 mm and a length of 900 mm. The catalyst was tested for isomerization of 1-butene to 2-butene at reaction conditions of a temperature of 60 °C, a WHSV of total feed of 1.5/hr, and a pressure of 6 barg. The commercial feed for the reaction was 0.1 mol% of ethane, 3.5 mol% of propane, 18.3 mol% of isobutene, 17.3 mol% of n-butane, 60.3 mol% of 1-butene, 0.1 mol% of trans 2-butene, and 0.4 mol% of cis -2-butene. At the above reaction conditions, the resulting steady product composition was 12.6 mol% of 1-butene, 21.4 mol% of trans 2-butene, 25.8 mol% of cis 2-butene, and about 40.2 mol% of inert. At a low reaction temperature (e.g., 25°C-100°C), K/Al₂O₃ catalyst was active for isomerization of 1-butene to 2-butene and stable for almost 1 month at WHSV of 0.5/hr with respect to total butenes in feedstock.

### Example 5

The catalyst 5 wt.%Re₂O₇/γ-Al₂O₃ is prepared by incipient wetness impregnation of the support with a HReO₄ solution. About 1.54 g of HReO₄ was dissolved in about 10.6 ml of DI water. The pore volume of γ-Al₂O₃ is 0.6, which means that 0.6 grams of water is necessary per gram of support for the incipient wetness method to disperse metal uniformly. Therefore, the total water required for 19 grams of support is 11.4 grams. However, 0.77 grams of water is contributed by the Rhenium precursor (HReO₄). Therefore, the necessary amount of water to be added is 10.6 grams for 19 grams of support.

The impregnation was conducted by contacting about 19 grams of calcined spherical alumina support with the HReO₄ solution at room temperature. The impregnated alumina support was then kept at room temperature for 2 hours and dried at 120 °C for 16 hours. The dried alumina support was then calcined at 550 °C for 5 hours at a heating rate of 5 °C/min in the presence of air in a down flow tubular reactor. After calcination, the catalyst was cooled in the continued presence of air. The catalyst was stored in an airtight container. The final calculated composition of the catalyst is 5wt. % Re₂O₇/Al₂O₃. The catalyst was tested under similar conditions as described in Example 2 and the results are shown in FIG. 6. FIG. 6 is a graphical representation of the isomerization reaction using 5 wt. % Re₂O₇/γ-Al₂O₃ under the reaction conditions: WHSV 0.5 /hr at a reaction temperature of about 300 °C and 1 atmospheric pressure.

When ranges are disclosed herein, ranges from any lower limit may be combined with any upper limit to recite a range not explicitly recited, as well as, ranges from any lower limit may be combined with any other lower limit to recite a range not explicitly recited, in the same way, ranges from any upper limit may be combined with any other upper limit to recite a range not explicitly recited. Additionally, reference to values stated in ranges includes each and every value within that range, even though not explicitly recited. Thus, every point or individual value may serve as its own lower or upper limit combined with any other point or individual value or any other lower or upper limit, to recite a range not explicitly recited.

Other obj ects, features and advantages of the disclosure will become apparent from the foregoing drawings, detailed description, and examples. These drawings, detailed description, and examples, while indicating specific embodiments of the disclosure, are given by way of illustration only and are not meant to be limiting. In further embodiments, features from specific embodiments may be combined with features from other embodiments. For example, features from one embodiment may be combined with features from any of the other embodiments. In further embodiments, additional features may be added to the specific embodiments described herein. It should be understood that although the disclosure contains certain aspects, embodiments, and optional features, modification, improvement, or variation of such aspects, embodiments, and optional features can be resorted to by those skilled in the art, and that such modification, improvement, or variation is considered to be within the scope of this disclosure.

## Claims

1. A method for olefin bond isomerization in the presence of a γ-alumina-based catalyst, the method comprising:
heating a γ-alumina-based catalyst in an isomerization reactor to a temperature ranging from about 450 °C to about 550 °C to produce an activated γ-alumina-based catalyst, wherein the activated γ-alumina-based catalyst contains either an alkali metal hydroxide ranging from 5 weight percent (wt.%) to 10 wt.% or rhenium oxide ranging from 3 wt.% to 8 wt.%;
cooling the activated γ-alumina-based catalyst to a temperature below 450°C; and
supplying an aliphatic light internal olefin-rich stream to the isomerization reactor to convert a portion of the aliphatic light internal olefin-rich stream to an aliphatic α-olefin-rich stream.

2. The method according to claim 1, wherein the method is a method for the isomerization of 2-butene to 1-butene, and wherein the activated γ-alumina-based catalyst has a surface area ranging from about 175 to 250 square meters per gram.

3. The method according to claim 1 or claim 2, wherein the alkali metal hydroxide is a potassium hydroxide.

4. The method according to any one of claims 1-3, wherein the isomerization reactor is a tubular fixed bed reactor.

5. The method according to any one of claims 1-3, wherein the activated γ-alumina-based catalyst is cooled in the presence of nitrogen.

6. The method according to any one of claims 1-3, wherein the aliphatic light internal olefin-rich stream contains greater than 30 wt. % of 2-butenes.

7. A method for preparing a γ-alumina-based catalyst, the method comprising:
calcining a γ-alumina-based support at a temperature ranging from about 450 °C to about 550 °C to produce a calcined γ-alumina-based support;
treating the calcined γ-alumina-based support with an aqueous alkali metal hydroxide solution or an aqueous perrhenic acid solution to form an impregnated γ-alumina-based support;
drying the impregnated γ-alumina-based a support at a temperature ranging from about 80 °C to about 120 °C; and
calcining the impregnated alumina support at a temperature ranging from about 450 °C to about 550 °C to produce the γ-alumina-based catalyst.

8. The method according to claim 7, wherein the calcined γ-alumina-based support is treated with an aqueous alkali metal hydroxide solution, and wherein the aqueous alkali metal hydroxide solution is sprayed over the γ-alumina-based support in the rotary-type impregnator unit at a gauge pressure of about 1.5 barg to about 4 barg.

9. The method according to any one of claims 7-8, wherein the calcined γ-alumina-based support is treated with an aqueous alkali metal hydroxide solution, and wherein the amount of the alkali metal hydroxide in the γ-alumina-based catalyst ranges from 5 wt.% to 10 wt.%.

10. The method according to claim 9, wherein the calcined γ-alumina-based support is treated with an aqueous alkali metal hydroxide solution, and wherein the aqueous alkali metal hydroxide solution contains potassium hydroxide with a molar concentration ranging from about 4M to about 8M.

11. The method according to any one of claims 7-10, wherein the γ-alumina-based support contains γ-alumina-based spheres with a surface area ranging from about 175 m²/g to about 250 m²/g.

12. The method according to any one of claims 7-12, wherein the γ-alumina-based support contains γ-alumina-based spheres with a pore volume ranging from about 0.5 milliliter per gram (mL/g) to about 0.75 mL/g.

13. The method according to claim 7, wherein the calcined γ-alumina-based support is treated with an aqueous perrhenic acid solution, and wherein the aqueous perrhenic acid solution is sprayed over the γ-alumina-based support in the rotary-type impregnator unit at a gauge pressure of about 2 barg.

14. The method according to any one of claims 7 or 13, wherein the γ-alumina-based catalyst contains rhenium oxide ranging from 3 wt.% to 8 wt.%.

15. The method according to any one of claims 13-14, wherein the γ-alumina-based support contains γ-alumina-based spheres with a surface area ranging from about 175 m²/g to about 250 m²/g and a pore volume ranging from about 0.5 mL/g to about 0.75 mL/g.
